# EUROPEAN PATENT SPECIFICATION

(11) **EP 4 213 819 B1**
(45) Date of publication and mention of the grant of the patent: **10.07.2024**
(21) Application number: 21777517.0
(22) Date of filing: 15.09.2021
(51) Int. Cl.: A61K 31/122, A61K 36/38, A61N 5/06, A61P 31/14

(54) **VIRICIDAL COMPOSITION AND USES THEREOF**
VIRIZIDE ZUSAMMENSETZUNG UND VERWENDUNGEN DAVON
COMPOSITION VIRICIDE ET SES UTILISATIONS

(30) Priority: 16.09.2020 EP 20382817
(43) Date of publication of application: 26.07.2023
(73) Proprietor: Dermo Magister, S.L., 28221 Madrid (ES); Universidad Autónoma de Madrid, 28049 Madrid (ES); Fundación Instituto de Investigación Sanitaria Aragón, 50009 Zaragoza (ES)
(72) Inventor: GONZÁLEZ RODRÍGUEZ, Salvador, 28221 Madrid (ES); JUARRANZ DE LA FUENTE,, Ángeles, 28049 Madrid (ES); GILABERTE CALZADA, Yolanda, 50009 Zaragoza (ES); LÓPEZ, José Antonio, 28049 Madrid (ES)
(74) Representative: Clarke, Modet y Cía., S.L.
(86) International application number: PCT/EP2021/075288
(87) International publication number: WO 2022/058332

(56) References cited:
- CN-A- 101 744 794
- US-A1- 2004 137 088
- CHEN HUIJIE ET AL: "Antiviral Activity Against Infectious Bronchitis Virus and Bioactive Components of Hypericum perforatum L.", FRONTIERS IN PHARMACOLOGY, vol. 10, 29 October 2019 (2019-10-29), XP055781524, DOI: 10.3389/fphar.2019.01272
- HUDSON J B ET AL: "Antiviral activities of hypericin", ANTIVIRAL RESEARCH, ELSEVIER BV, NL, vol. 15, no. 2, 1 February 1991 (1991-02-01), pages 101 - 112, XP023702905, ISSN: 0166-3542, [retrieved on 19910201], DOI: 10.1016/0166-3542(91)90028-P
- SMITH MICHOLAS ET AL: "Repurposing Therapeutics for COVID-19: Supercomputer-Based Docking to the SARS-CoV-2 Viral Spike Protein and Viral Spike Protein-Human ACE2 Interface", 11 March 2020 (2020-03-11), pages 1 - 31, XP055781090, Retrieved from the Internet <URL:https://s3-eu-west-1.amazonaws.com/itempdf74155353254prod/11871402/Repurposing_Therapeutics_for_COVID-19__Supercomputer-Based_Docking_to_the_SARS-CoV-2_Viral_Spike_Protein_and_Viral_Spike_v4.pdf> [retrieved on 20210302], DOI: 10.26434/chemrxiv.11871402.v4

## Description

The present invention relates to the field of viricidal products, more precisely products against coronavirus, even more precisely, products to eliminate coronavirus from surfaces and/or to prevent contamination of surfaces by coronavirus.

Cleaning and disinfection of surfaces (including the skin) and objects has been a concern since long time ago in several industries, for example, alimentary industry, pharmaceutical industry and hospitals, to avoid contaminations and infections.

However, with the outbreak of COVID-19 in December of 2019 (viral infection caused by Coronavirus SARS-CoV-2) and its subsequent spread all around the world, the need to clean and disinfect surfaces and the skin (mainly of hands), to ensure elimination and/or inactivation of said virus and, hence, prevent infection, has extended to all the industries and to all the aspects of our daily activity. As it is widely known, one of the main recommendation made by the World Health Organisation (and by most of the national governments) to fight against COVID-19 disease is the appropriate and frequent washing of hands by means of soap or other commercially available products, such as, hydroalcoholic gels; and the appropriate and frequent washing and disinfection of working or shared areas.

Nowadays, there are a range of products for the cleaning and disinfection of the skin and/or of inert surfaces to ensure that Coronavirus SARS-CoV-2 is effectively eliminated and/or inactivated and, hence, prevent its infection and COVID-19 disease. Examples thereof are soaps, bleach, hydroalcoholic gels or solutions, or hydroxide peroxide.

However, said products are normally aggressive with the skin and/or with the inert surfaces to which they are applied. Therefore, it would be desirable to have alternative products which ensure the elimination of coronavirus (preferably, Coronavirus SARS-CoV-2) from surfaces (including skin) and/or which prevent colonization or contamination of surfaces by coronavirus (preferably, Coronavirus SARS-CoV-2) and which are less aggressive.

The inventors of the present invention, after extensive and exhaustive research, have surprisingly found that a composition comprising hypericin (preferably, a composition comprising hypericum extract) in combination with light allows for the effective elimination and/or inactivation of coronavirus from surfaces, including skin, or from liquids. The combination of said hypericin (preferably, hypericum extract) with light, as it is directly derivable form the examples included below, provides for a synergistic effect in the elimination and/or inactivation of coronavirus. In addition, its activity remains in the surface or liquid were the treatment has been applied and allows also the prevention of viral contamination of said surface or liquid by coronavirus. The present invention is less aggressive that the options available in the state of the art as it refers to the combination of a compound derived from a plant extract with light (preferably, visible light).

Hence, the present invention solves the technical problems present in the state of the art and mentioned above. The invention is defined in the appended claims.

In a first aspect the present invention refers to the combined use of light and a composition comprising hypericin (preferably, a composition comprising hypericum extract) in the prevention and/or treatment of a coronavirus disease.

In a second aspect, the present invention refers to the use of a composition comprising hypericin (preferably, a composition comprising hypericum extract) for the manufacture of a medicament for the treatment and/or prevention of a coronavirus disease, wherein the composition is applied on the skin of a subject and after its application is exposed to light.

In a third aspect, the present invention refers to the treatment and/or prevention of a coronavirus disease in a subject in need thereof, comprising the application of a composition comprising hypericin (preferably, a composition comprising hypericum extract) on the skin of the subject in need of the treatment and/or prevention; and exposing the skin with the composition to light.

In a fourth aspect, the present invention refers to the combined use of light and a composition comprising hypericin (preferably, a composition comprising hypericum extract) for the inactivation of coronavirus from a surface (preferably, an inert surface, including face and surgical masks, googles and individual protection equipment) or a liquid.

In a fifth aspect, the present invention refers to the combined use of light and a composition comprising hypericin (preferably, a composition comprising hypericum extract) for the prevention of viral contamination of a surface (preferably, an inert surface, including face and surgical masks, googles and individual protection equipment) or a liquid by coronavirus.

In an additional aspect, the present invention refers to a method for the inactivation of coronavirus from a surface (preferably, an inert surface, including face and surgical masks, googles and individual protection equipment) or a liquid based in the application of a composition comprising hypericin (preferably, a composition comprising hypericum extract) to the surface or the liquid; and exposing said surface or liquid with the composition to light.

In a seventh aspect, the present invention refers to a method for the prevention of viral contamination of a surface (preferably, an inert surface, including face and surgical masks, googles and individual protection equipment) or a liquid by coronavirus based in the application of a composition comprising hypericin (preferably, a composition comprising hypericum extract) to the surface or the liquid; and exposing said surface or liquid with the composition to light.

In a further aspect, the present invention refers to the use of a composition comprising hypericin (preferably, a composition comprising hypericum extract) in combination with light for the cosmetic cleaning and/or hygienization of the skin of a subject.

In a final aspect, the present invention refers to a method for the cosmetic cleaning and/or hygienization of skin of a subject, comprising: application of a composition comprising hypericin (preferably, a composition comprising hypericum extract) to the skin; and exposing said skin with the composition to light.

As used herein, "visible light" and its plural refer to light of between 420 and 700 nm. Said visible light, as used herein can be light of a single wavelength or of a combination of wavelengths.

As used herein, "exposed" and "irradiated", when referring to light are used interchangeably and acquire the meaning they commonly have in the state of the art, this is, that light effectively reaches the objective.

Therefore, as noted above, in a first aspect, the present invention refers to a composition comprising hypericin for use in the prevention and/or treatment of a coronavirus disease wherein the composition is used in combination with light.

In a preferred embodiment, the composition is applied on the skin of a subject and subsequently exposed to light. More preferably, the composition is topically applied on the skin of the subject.

The composition comprising hypericin can be in any form known in the state of the art, more preferably, in any form known in the state of the art which is suitable for application in the skin, more preferably in the form of a liquid, a hydrogel, a cream (for example, a therapeutic cream, a solar cream or a cosmetic cream), an aerosol or a spray.

It is contemplated that the subject is an animal or a human, more preferably, the subject can be a mammal, a bird or a human, even more preferably, the subject is a human.

In the composition, hypericin is preferably at a concentration of at least 0.00075 µg/mL, more preferably at least 0.0015 µg/mL, more preferably at least 0.00225 µg/mL, more preferably at least 0.003 µg/mL, even more preferably at least 0.006 µg/mL. Therefore, in a preferred embodiment, the concentration of hypericin in the composition is between 0.00075 µg/mL and 0.02 µg/mL, preferably between 0.00075 µg/mL and 0.004 µg/mL, more preferably 0.003 µg/mL. In a most preferred embodiment, the concentration of hypericin in the composition is between 0.00225 µg/mL and 0.02 µg/mL, more preferably between 0.003 µg/mL and 0.02 µg/mL, even more preferably 0.006 µg/mL.

The hypericin can be obtained by any means known in the state of the art, for example, chemical synthesis or extraction from plants.

In the most preferred embodiment, hypericin is provided in the composition in the form of an hypericum extract which comprises hypericin. Therefore, in this most preferred embodiment, the composition comprises an hypericum extract.

Preferably, the hypericum extract is an extract from *Hypericum perforatum,* more preferably, an extract obtained from any part of *Hypericum perforatum,* more preferably, an extract obtained from the rhizome, leaves, grass or combinations thereof of *Hypericum perforatum,* more preferably, an extract from the leaves or grass of *Hypericum perforatum,* even more preferably an extract from dry leaves of *Hypericum perforatum.*

Preferably, the hypericum extract is obtained by means of extraction with water:ethanol, more preferably, by using 8 parts of water and 1 part of ethanol. As noted above, the extraction is preferably performed to *Hypericum perforatum* (which is as noted in the previous paragraph).

The hypericum extract has, preferably, a concentration of hypericin of between 0.15% (weight/volume, hereinafter w/v) and 0.40% (w/v), more preferably 0.30% (w/v).

In addition, preferably, the concentration of hypericum extract in the composition is 0.5 µg/mL, more preferably, at least 1 µg/mL, more preferably at least 1.5 µg/mL, even more preferably, at least 2 µg/mL. Therefore, in a preferred embodiment, the concentration of hypericum extract in the composition is between 0.5 µg/mL and 5 µg/mL, preferably between 0.5 µg/mL and 1 µg/mL, more preferably 1 µg/mL. In a most preferred embodiment, the concentration of hypericum extract in the composition is between 1.5 µg/mL and 5 µg/mL, more preferably between 2 µg/mL and 5 µg/mL, even more preferably 2 µg/mL.

It is contemplated that the light used in this first aspect of the present invention is any light. In addition, it is contemplated that said light can be natural light, artificial light or combinations thereof. Said light can be provided by any means known in the state of the art.

Therefore, in a preferred embodiment, the light is natural light.

In another preferred embodiment, the light is artificial light provided by means of a lamp. Said lamp, preferably, has a potency of between 50 and 150 mW/cm², even more preferably, 90 mW/cm²,

In a preferred embodiment the light comprises visible light, more preferably, the light is visible light. Said visible light, as noted above, can be of a single wavelength or of a combination of wavelengths, more preferably, of a combination of wavelengths.

It is contemplated that the used visible light is selected from blue light (420-495 nm), amber light (550-580 nm), white light (420-700 nm) or combinations thereof.

In the most preferred embodiment, visible light is white light (420-700 nm).

With regard to the dose of light applied, it is preferably at least 10 J/cm², more preferably between 10 and 36 J/cm², more preferably between 15 and 36 J/cm², even more preferably 15 J/cm².

As it is readily understandable by a person skilled in the art, the time of exposure will depend on the source of light and the desired dose. Therefore, a person skilled in the art will be able to determine and adapt the time of exposure on the basis of the source of light used and the required dose. For example, for a lamp of 90 mW/cm², a dose of 15 J/cm² will correspond to 2 minutes and 30 seconds of irradiation or exposure.

The present invention can be used for the prevention and/or treatment of any coronavirus disease.

In a preferred embodiment, the coronavirus disease is a disease caused by Coronavirus HCoV-229E. Therefore, in this preferred embodiment, the present invention is for use in the prevention and/or treatment of a disease caused by Coronavirus HCoV-229E, even more preferably, in the prevention of a disease caused by Coronavirus HCoV-229E.

In a most preferred embodiment, the present invention is for use in the prevention and/or treatment of COVID-19, even more preferably, in the prevention of COVID-19.

As noted above, COVID-19 is caused by Coronavirus SARS-CoV-2 and, therefore, preferably, the present invention in this most preferred embodiment is for the treatment and/or prevention of the disease caused by Coronavirus SARS-CoV-2, more preferably, for the prevention of the disease caused by Coronavirus SARS-CoV-2.

Said treatment and/or prevention of COVID-19 (preferably, prevention of COVID-19) is preferably carried out by means of inactivation of Coronavirus SARS-CoV-2 from the skin of the subject, more preferably from the skin of the hands of the subject.

As it is apparent from the examples included below and from the whole explanation provided herein, the present invention is useful for the elimination or inactivation of coronavirus (preferably, Coronavirus HCoV-229E or Coronavirus SARS-CoV-2). Therefore, the present invention is useful for the elimination and/or inactivation of coronavirus from the skin of subjects susceptible of being infected preventing them from being infected by said coronavirus. In addition, due to the nature of the invention (the combination of a compound obtained from a plant extract with light), it is less aggressive than the options available in the state of the art to eliminate and/or inactivate coronavirus from the skin of subjects and, therefore, is better tolerated, allowing for repeated application or use of the invention without adverse effects. Hence, solving, the problems present in the state of the art and mentioned above.

In a second aspect, the present invention refers to the use of a composition comprising hypericin for the manufacture of a medicament for the treatment and/or prevention of a coronavirus disease, wherein the composition is applied on the skin of a subject and after its application is exposed to light.

The composition comprising hypericin, the hypericin, the coronavirus disease, the subject, the skin and the light are as already defined above in the first aspect of the present invention.

In a third aspect, the present invention refers to a method for the treatment and/or prevention of a coronavirus disease in a subject in need thereof, comprising:
a) topical application of a composition comprising hypericin on the skin of the subject in need of the treatment and/or prevention; and
b) exposing the skin with the composition to light.

The composition comprising hypericin, the hypericin, the coronavirus disease, the subject, the skin and the light are as already defined above in the first aspect of the present invention.

In a fourth aspect, the present invention refers to the use of a composition comprising hypericin for the inactivation of coronavirus from a surface or a liquid, wherein the composition is applied on said surface or said liquid and after its application is exposed to light.

The composition comprising hypericin, the hypericin and the light are as already explained above in the first aspect of the present invention.

In this fourth aspect of the present invention, the surface is, preferably, an inert surface, this is, the surface of an inert object, more preferably, the surface of a facemask, of a surgical mask, of googles or of individual protection equipment.

It is contemplated that the liquid is any liquid known or available in the state of the art. Preferably, the liquid is blood or plasma, more preferably, human blood or human plasma.

The use of this fourth aspect of the present invention is carried out ex vivo, more preferably, the use of this fourth aspect of the present invention is carried out in vitro.

The composition comprising hypericin, as noted above, is as explained in the first aspect of the present invention, more preferably is in the form of a liquid, an aerosol or a spray.

It is contemplated that the coronavirus is any coronavirus known in the state of the art (and any coronavirus discovered in the future). Preferably, the coronavirus is Coronavirus HCoV-229E or Coronavirus SARS-CoV-2, more preferably, Coronavirus SARS-CoV-2.

As noted above and as it is apparent from the examples included below, the present invention is useful for the elimination or inactivation of coronavirus (preferably, Coronavirus HCoV-229E or Coronavirus SARS-CoV-2) and, hence, it can be used to eliminate and/or inactivate said virus in surfaces (preferably of inert objects) or in liquids to, therefore, disinfect them and prevent the spread of the virus.

Also within the scope of the present invention are inert objects comprising in their surface the composition comprising hypericin (which is as explained above in the first aspect of the present invention). The inert object is as explained above in the fourth aspect of the present invention.

In a fifth aspect, the present invention refers to the use of a composition comprising hypericin for the prevention of viral contamination of a surface or a liquid by coronavirus.

The composition comprising hypericin, the hypericin and the light are as already explained above in the first aspect of the present invention. The surface, the liquid and the coronavirus are as explained in the fourth aspect of the present invention.

The use of this fifth aspect of the present invention is carried out ex vivo, more preferably, the use of this fifth aspect of the present invention is carried out in vitro.

The composition comprising hypericin, as noted above, is as explained in the first aspect of the present invention, more preferably is in the form of a liquid, an aerosol or a spray.

As noted above and as demonstrated in the examples included below the present invention is useful for the elimination or inactivation of coronavirus (preferably, Coronavirus HCoV-229E or Coronavirus SARS-CoV-2). In addition, it has been seen that that the viricidal activity would remain in the treated surface, hence, allowing the prevention of the contamination of said surface by coronavirus.

In a sixth aspect, the present invention refers to a method for the inactivation of coronavirus from a surface or a liquid comprising:
a) application of a composition comprising hypericin to the surface or the liquid; and
b) exposing said surface or said liquid with the composition to light.

In this sixth aspect of the present invention, the composition comprising hypericin, the hypericin and the light are as already explained above in the first aspect of the present invention; and the surface, the liquid and the coronavirus are as explained in the fourth aspect of the present invention.

The method of this sixth aspect of the present invention is carried out ex vivo, more preferably, the method of this sixth aspect of the present invention is carried out in vitro.

The composition comprising hypericin, as noted above, is as explained in the first aspect of the present invention, more preferably is in the form of a liquid, an aerosol or a spray.

The technical effects noted above, especially for the fourth aspect of the present invention are directly applicable to this sixth aspect of the present invention.

In a seventh aspect, the present invention refers to a method for the prevention of viral contamination of a surface or a liquid by coronavirus comprising:
a) application of a composition comprising hypericin to the surface or the liquid; and
b) exposing said surface or said liquid with the composition to light.

In this seventh aspect of the present invention, the composition comprising hypericin, the hypericin and the light are as already explained above in the first aspect of the present invention; and the surface, the liquid and the coronavirus are as explained in the fourth aspect of the present invention.

The method of this seventh aspect of the present invention is carried out ex vivo, more preferably, the method of this seventh aspect of the present invention is carried out in vitro.

The composition comprising hypericin, as noted above, is as explained in the first aspect of the present invention, more preferably is in the form of a liquid, an aerosol or a spray.

The technical effects noted above, especially for the fifth aspect of the present invention are directly applicable to this seventh aspect of the present invention.

In an eighth aspect of the present invention, the present invention refers to the use of a composition comprising hypericin for the cosmetic cleaning and/or hygienization of the skin of a subject, wherein the composition is applied on the skin of the subject and after its application is exposed to light.

In this eighth aspect of the present invention, the composition comprising hypericin, the hypericin, the skin, the light and the subject are as already explained above in the first aspect of the present invention.

The cosmetic cleaning and/or hygienization of the skin is, preferably, for the inactivation of coronavirus from the skin, even more preferably, from the skin of the hands. The coronavirus is, preferably, Coronavirus HCoV-229E or Coronavirus SARS-CoV-2, more preferably, Coronavirus SARS-CoV-2.

The cosmetic cleaning and/or hygienization of the skin also includes the prevention of the viral contamination of the skin by coronavirus, preferably, of the skin of the hands. The coronavirus is as already explained above.

In this eighth aspect of the present invention, in a preferred embodiment, the composition is in the form of a liquid, hydrogel, spray or aerosol. In another preferred embodiment, the composition is in the form of a cosmetic cream or a cosmetic liquid solution, more preferably of a topical photoprotectant (this is, incorporating or comprising other cosmetic active principles, preferably at least one solar filter).

As noted above and as it is apparent from the examples included below, the present invention is useful for the elimination or inactivation of coronavirus (preferably, Coronavirus SARS-CoV-2) and, hence, provides for the required activity for the appropriate cosmetic cleaning and/or hygienization of the skin.

In a final aspect, the present invention refers to a method for the cosmetic cleaning and/or hygienization of the skin of a subject, comprising:
a) application of a composition comprising hypericin to the skin of the subject; and
b) exposing said skin with the composition to light.

In this final aspect of the present invention, the composition comprising hypericin, the hypericin, the subject and the light are as already explained above in the first aspect of the present invention.

On its turn, the cosmetic cleaning and/or hygienization of the skin are as explained above in the eighth aspect of the present invention.

In this final aspect of the present invention, in a preferred embodiment, the composition is in the form of a liquid, hydrogel, spray or aerosol. In another preferred embodiment, the composition is in the form of a cosmetic cream or a cosmetic liquid solution, more preferably of a topical photoprotectant (this is, incorporating or comprising other cosmetic active principles, preferably at least one solar filter).

Also, the technical effects mentioned above (especially those of the eighth aspect), are applicable to this final aspect.

To allow a better understanding, the present invention is described in more detail below with reference to the enclosed figures, which are presented by way of example, and with reference to illustrative and non-limitative examples.

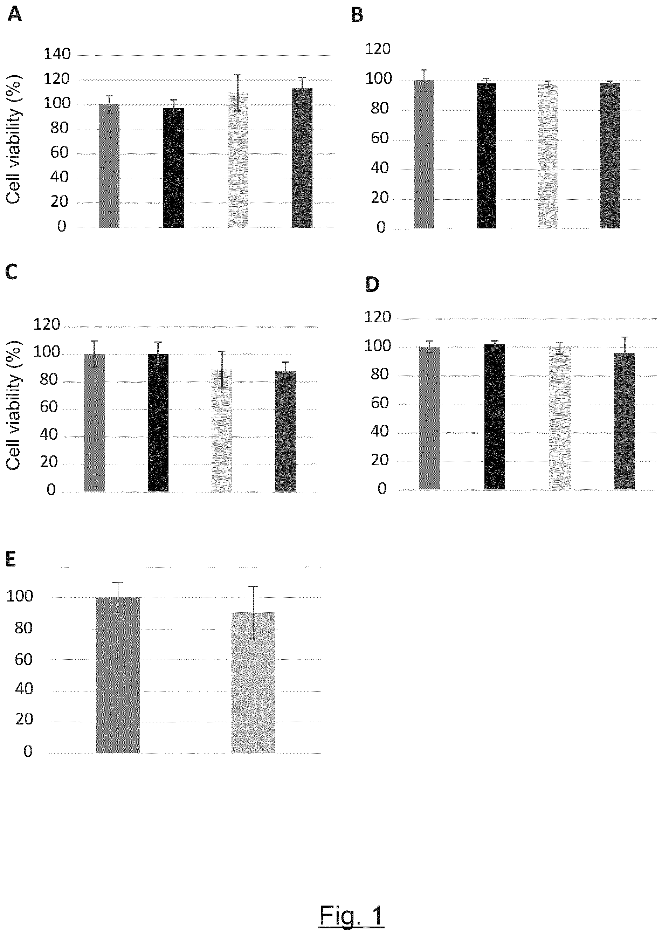

Figure 1 shows effects on cell viability induced by Hypericum extract (H) and Methylene blue (MB) subjected or not to white light irradiation (420-700 nm, 90 mW/cm²) at a dose of 15 J/cm², as well as the effects on cell viability of the irradiation with said white light alone. Cell viability was evaluated by means of the MTT test (the MTT assay is a colorimetric test for measuring cell metabolic activity based on the ability of cellular enzymes to reduce the tetrazolium salt (3-(4,5-dimethylthiazol-2-yl)-2,5-diphenyltetrazolium bromide, MTT, to its insoluble formazan with a purple colour by metabolically active cells) (see example 1 included below). Figure 1A shows Huh-7 hepatocarcinoma cell line (hereinafter, Huh7 cells) wherein the different treatment groups showed therein were not irradiated with white light. The bars on the x-axis of figure 1A show the different treatment groups which are, from left to right: control (no treatment); methylene blue (0,25 µg/ml in DMEM with 2% (volume/volume, v/v) FBS (Foetal Bovine Serum)); Hypericum extract at 1 µg/ml in DMEM with 2% (v/v) FBS; and Hypericum extract at 2 µg/ml in DMEM with 2% (v/v) FBS. Figure 1B shows Vero cells wherein the different treatment groups showed therein were not irradiated with white light. The bars on the x-axis of figure 1B show the different treatment groups which are, from left to right: control (no treatment); methylene blue (0,25 µg/ml in DMEM with 2% (v/v) FBS (Foetal Bovine Serum)); Hypericum extract at 1 µg/ml in DMEM with 2% (v/v) FBS; and Hypericum extract at 2 µg/ml in DMEM with 2% (v/v) FBS. Figure 1C shows Huh7 cells wherein the different treatment groups showed therein were irradiated with white light. The bars on the x-axis of figure 1C show the different treatment groups which are, from left to right: control (no treatment); methylene blue (0,25 µg/ml in DMEM with 2% (v/v) FBS (Foetal Bovine Serum)); Hypericum extract at 1 µg/ml in DMEM with 2% (v/v) FBS; and Hypericum extract at 2 µg/ml in DMEM with 2% (v/v) FBS. Figure 1D shows Vero cells wherein the different treatment groups showed therein were irradiated with white light. The bars on the x-axis of figure 1D show the different treatment groups which are, from left to right: control (no treatment); methylene blue (0,25 µg/ml in DMEM with 2% (v/v) FBS (Foetal Bovine Serum)); Hypericum extract at 1 µg/ml in DMEM with 2% (v/v) FBS; and Hypericum extract at 2 µg/ml in DMEM with 2% (v/v) FBS. Figure 1E shows the effect on Huh7 cell viability of irradiation with white light. The bars on the x-axis of figure 1E show the different treatment groups which are, from left to right: control (no treatment); and cells irradiated with white light (420-700 nm, 90 mW/cm²) at a dose of 15 J/cm². In figures 1A to 1E, the y-axis shows or refers to cell viability reflected as percentage (stablishing as 100% of cell viability the control untreated cells and, then, on said basis, calculating the percentage of cell viability the rest of the groups). In figures 1A to 1E each of the bars represents the mean of the percentage of cell viability ± standard deviation.

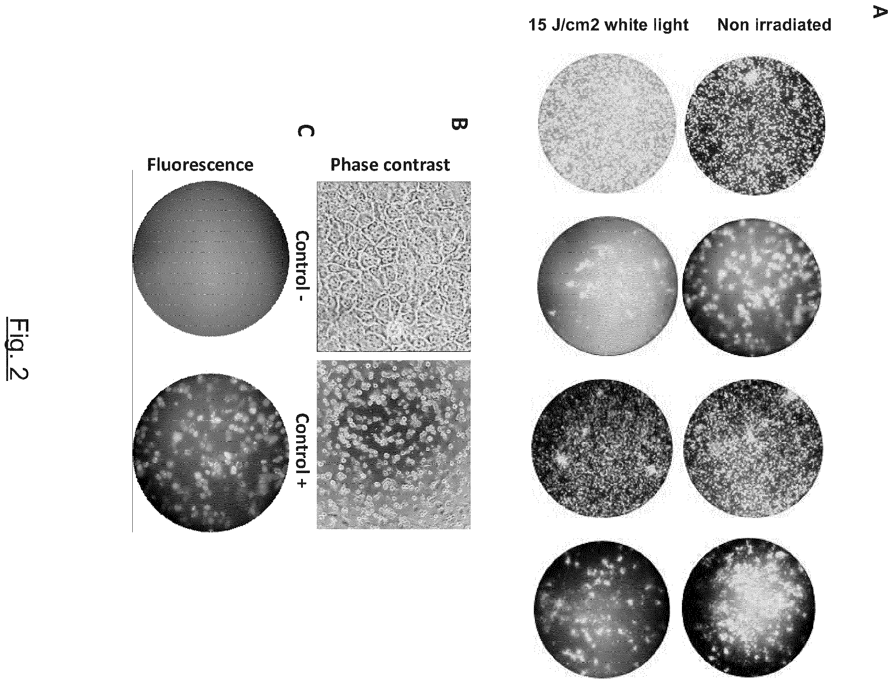

Figure 2 shows Coronavirus HCoV-229E-GFP infectivity in Huh7 cells under different treatment conditions and at a multiplicity of infection (MOI) of 0.5 (see Example 2). Figure 2A shows fluorescence images of the infectivity of Coronavirus HCoV-229E-GFP in Huh7 cells in different conditions tested in example 2. In figure 2A, the first line of images shows non-irradiated samples and the second line of images shows samples irradiated with white light (420-700 nm, 90 mW/cm²) at a dose of 15 J/cm² (where applicable, the irradiation was performed to the viral HCoV-229E-GFP sample prior to cell infection, as explained in example 2). Also in figure 2A, both in the first and second lines of images, the columns show the different treatment groups, which are, from left to right: control (infected cells with non-treated virus); cells infected with virus treated with methylene blue (0,25 µg/ml in DMEM with 2% (v/v) FBS (Foetal Bovine Serum)); cells infected with virus treated with Hypericum extract at 1 µg/ml in DMEM with 2% (v/v) FBS; and cells infected with virus treated with Hypericum extract at 2 µg/ml in DMEM with 2% (v/v) FBS. In figure 2A, cellular infection was performed for one hour and then cells were washed with Phosphate Buffered Saline (NaCl, 0,137 M; KCI, 0,0027 M; Na₂HPO₄, 0,01 M; KH₂PO₄, 0,0018 M, PH 7.4) (hereinafter, PBS) and post-incubated in DMEM with 10% (v/v) FBS for 24 hours. Infection was evaluated by fluorescence microscopy since HCoV-229E-GFP shows the Green Florescent Protein (GFP); a high fluorescence is indicative of a high infection. Figure 2B shows phase contrast microscopy images wherein the left image shows non-infected Huh7 cells (and with no treatment, nor irradiation) and the right image shows Huh7 cells infected with HCoV-229E-GFP (wherein the virus has not been treated nor irradiated). Figure 2C shows fluorescence microscopy images wherein the left image shows non-infected Huh7 cells (and with no treatment, nor irradiation) and the right image shows Huh7 cells infected with HCoV-229E-GFP (wherein the virus has not been treated nor irradiated).

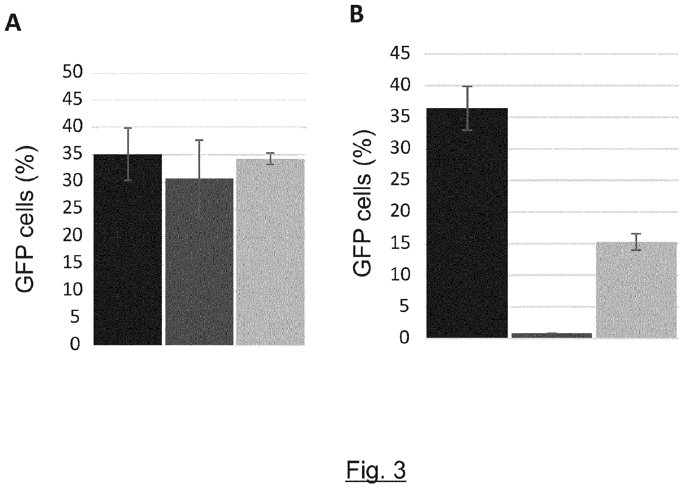

Figure 3 shows Coronavirus HCoV-229E-GFP infectivity in Huh7 cells under different treatment conditions and at a MOI of 0.5 (see Example 2), wherein infection was quantified by means of flow cytometry in a FACScalibur using an excitation wavelength of 436 nm to determine the amount of Green Fluorescent Protein (GFP). Figure 3A, refers to non-irradiated viral samples. In said figure 3A, the bars in the x-axis refer to or show the different treatment groups, from left to right: cells infected with non-treated and non-irradiated HCoV-229E-GFP; cells infected with virus treated with methylene blue (0,25 µg/ml in DMEM with 2% (v/v/) FBS (Foetal Bovine Serum); and non-irradiated); and cells infected with virus treated with Hypericum extract at 2 µg/ml in DMEM with 2% (v/v) FBS (and not irradiated). Figure 3B, refers to irradiated viral samples (with white light (420-700 nm, 90 mW/cm²) at a dose of 15 J/cm²). In said figure 3B, the bars in the x-axis refer to or show the different treatment groups, from left to right: cells infected with non-treated and irradiated HCoV-229E-GFP; cells infected with virus treated with methylene blue (0,25 µg/ml in DMEM with 2% (v/v) FBS (Foetal Bovine Serum); and irradiated); and cells infected with virus treated with Hypericum extract at 2 µg/ml in DMEM with 2% (v/v) FBS (and irradiated). Both in figures 3A and 3B, the y-axis refers to the % of cells with GFP.

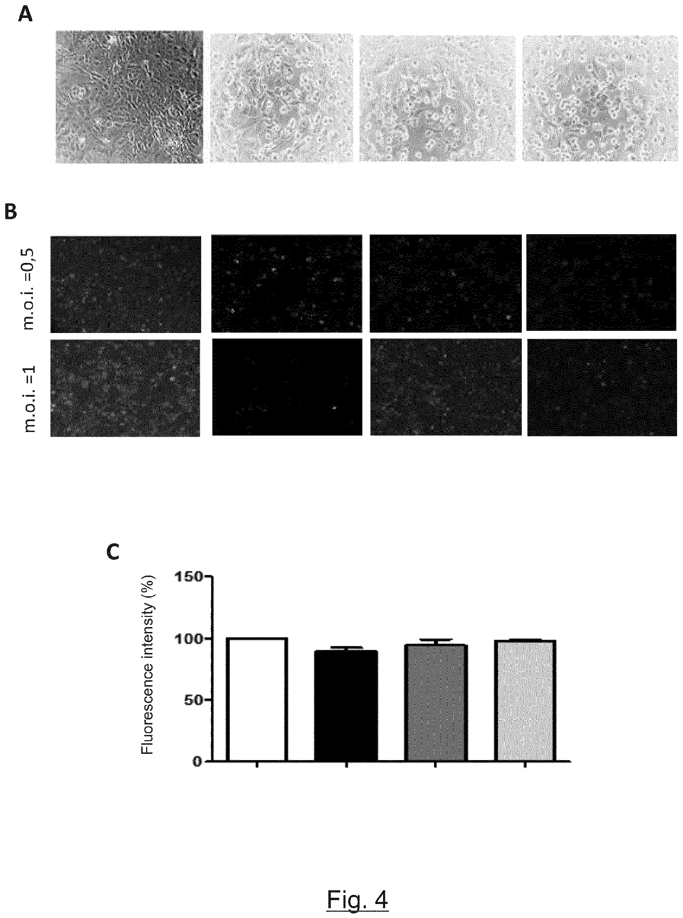

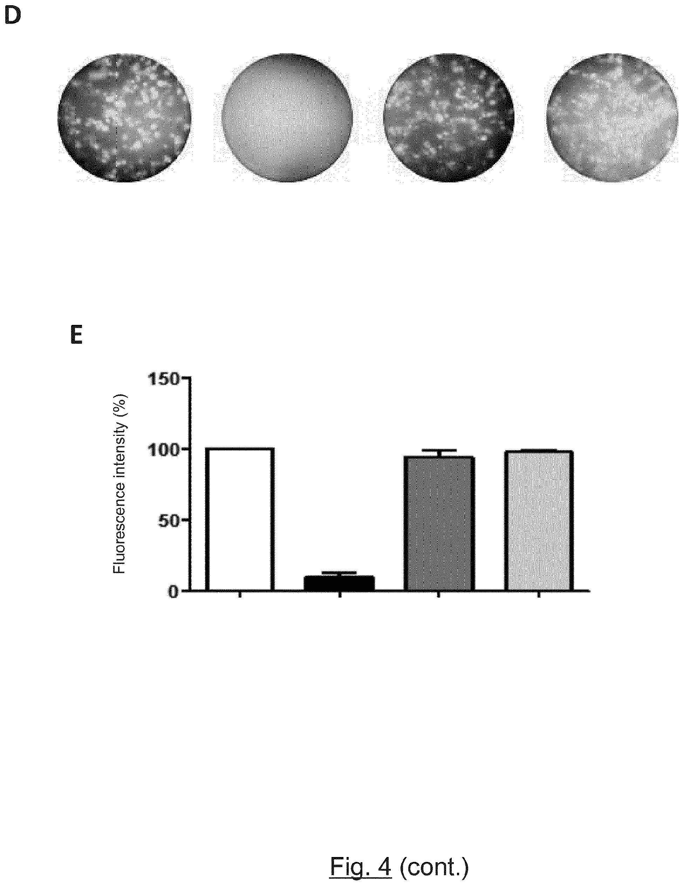

Figure 4 shows Herpes Simplex Virus type 1 HSV-1 K26-GFP infectivity in Vero cells under different treatment conditions and at a MOI of 0.5 or 1 (see Example 2). Figure 4A shows contrast phase microscopy images of the infectivity of HSV-1 K26-GFP in Vero cells in different conditions tested in example 2. Figure 4A images show, from left to right, non-infected Vero cells; Vero cells infected with HSV-1 K26-GFP virus not treated and not irradiated; Vero cells infected with HSV-1 K26-GFP virus treated with Hypericum extract at 1 µg/ml in DMEM with 2% (v/v) FBS and not irradiated; and Vero cells infected with HSV-1 K26-GFP virus treated with Hypericum extract at 2 µg/ml in DMEM with 2% (v/v) FBS and not irradiated, in all cases at a MOI of 1 (infected cells with HSV-1 K26-GFP at a MOI of 1, showed a rounded morphology). Figure 4B shows fluorescence microscopy images of the infectivity of HSV-1 K26-GFP in Vero cells in different conditions tested (non-irradiated) in example 2. In figure 4B, the first line of images shows a MOI of 0.5 and the second line shows a MOI of 1. Also in figure 4B, both in the first and second lines of images, the columns show different treatment groups, which are, from left to right: positive control (infected cells with non-treated and non-irradiated virus); cells infected with virus treated with methylene blue (0,25 µg/ml in DMEM with 2% (v/v) FBS (Foetal Bovine Serum) and non-irradiated); cells infected with virus treated with Hypericum extract at 1 µg/ml in DMEM with 2% (v/v) FBS (and not irradiated); and cells infected with virus treated with Hypericum extract at 2 µg/ml in DMEM with 2% (v/v) FBS (and not irradiated). In this figure 4B, Vero infected cells showed an intense fluorescence under blue excitation light and no differences were seen between virus exposed or not to the different treatments. Figure 4C shows a bar graph of the results observed in Figure 4B for the experiments performed with a MOI of 1. In this figure 4C, each of the bars in the x-axis refers to a treatment group, more precisely, from left to right: positive control (infected cells with non-treated and non-irradiated virus); cells infected with virus treated with methylene blue (0,25 µg/ml in DMEM with 2% (v/v) FBS (Foetal Bovine Serum) and non-irradiated); cells infected with virus treated with Hypericum extract at 1 µg/ml in DMEM with 2% (v/v) FBS (and not irradiated); and cells infected with virus treated with Hypericum extract at 2 µg/ml in DMEM with 2% (v/v) FBS (and not irradiated). In Figure 4C, the y-axis refers to the % of fluorescence intensity in comparison with that of the positive control (this is, stablishing the fluorescence intensity of the positive control as 100% and on the basis of this calculating the percentage of fluorescence intensity of the rest of the groups). Figure 4D shows fluorescence microscopy images of the infectivity of HSV-1 K26-GFP in Vero cells (MOI=1) in different conditions (in all of them being the virus irradiated with white light (420-700 nm, 90 mW/cm²) at a dose of 15 J/cm², tested in example 2, more particularly, images in figure 4D refer, from left to right to: positive control (cells infected with non-treated but irradiated virus); cells infected with virus treated with methylene blue (0,25 µg/ml in DMEM with 2% (v/v) FBS (Foetal Bovine Serum) and irradiated); cells infected with virus treated with Hypericum extract at 1 µg/ml in DMEM with 2% (v/v) FBS (and irradiated); and cells infected with virus treated with Hypericum extract at 2 µg/ml in DMEM with 2% (v/v) FBS (and irradiated). Figure 4E shows a bar graph of the results observed in Figure 4D. In this figure 4E, each of the bars in the x-axis refers to a treatment group, more precisely, from left to right: positive control (cells infected with non-treated but irradiated virus); cells infected with virus treated with methylene blue (0,25 µg/ml in DMEM with 2% (v/v) FBS (Foetal Bovine Serum) and irradiated); cells infected with virus treated with Hypericum extract at 1 µg/ml in DMEM with 2% (v/v) FBS (and irradiated); and cells infected with virus treated with Hypericum extract at 2 µg/ml in DMEM with 2% (v/v) FBS (and irradiated). In Figure 4E, the y-axis refers to the % of fluorescence intensity in comparison with that of the positive control (this is, stablishing the fluorescence intensity of the positive control as 100% and on the basis of this calculating the percentage of fluorescence intensity of the rest of the groups).

### EXAMPLES

### Example 1. In vitro analysis of the toxicity of a composition comprising hypericin (hypericum extract) and white light.

### Evaluation of the toxicity of the composition comprising hypericin (hypericum extract) and white light in cells.

### Cell types and treatment conditions.

Two human cell lines were used. These cell lines are usually employed to determine infective ability of the studied virus (Coronavirus, HCoV-229E; and Herpes simplex virus type 1, HSV-1 K26):
- Huh-7 hepatocarcinoma cell line (for Coronavirus) (hereinafter, Huh7 cells). This cell line was originally taken from a liver tumor in a 57-year-old Japanese male. The cell line is distributed by Japanese Cancer Research Resources Bank (JCRB) as JCRB0403. This cell line is normally used to determine infectivity of coronavirus.
- Vero cells obtained from African green monkey kidney (*Cercopithecus aethiops*) (for HSV-1) (hereinafter, Vero cells). The cell line was obtained from ATCC (CCL81). This cell line is normally used to determine infectivity of Herpes simplex virus.

Cells were cultured in Dulbecco's modified eagle medium (DMEM) supplemented with 1% (v/v) penicillin G (100 U/ml) and streptomycin (100 µg/ml) and 10% (v/v) fetal bovine serum (FBS) (HyClone Laboratories). Cells were maintained at 37°C, 5% humidity and 5% CO₂ in an incubator (Heraeus HERAcell, Thermo Scientific).

The hypericum extract (hereinafter, H) (Farmaquimica Sur) used comprised hypericin at a concentration of 0.30% in weight/volume (w/v), was used at two concentrations: 1 and 2 µg/ml in DMEM culture medium without Foetal Bovine Serum (FBS). Methylene blue (hereinafter, MB) (Sigma) was used as positive control (0.25 µg/ml in DMEN). Stock solutions were prepared in distilled water at a concentration of 10 mg/ml. The stocks were then diluted in phenol red-free DMEM with 2% (v/v) FBS to the desired final concentrations.

When cells, grown in 96-well tissue culture dishes (each well has a diameter of 5.5 mm), reached about a 60% of confluence, they were treated for 1 hour with 100 µL of H or MB and then were or not exposed to a source of white light (420-700 nm, 90 mW/cm²) at doses of 15 or 37 J/cm². The light source was placed under the culture plates so that cells were irradiated directly from below, avoiding any possible shielding effects exerted by the culture medium or the treatments. After irradiation, cells were washed three times with Phosphate Buffered Saline (NaCl, 0,137 M; KCI, 0,0027 M; Na₂HPO₄, 0,01 M; KH₂PO₄, 0,0018 M, PH 7.4) (hereinafter, PBS) and post-incubated in DMEM with 10% (v/v) FBS for 24 h.

Cell viability after treatments was determined using the MTT assay. MTT solution (Sigma) was added to cell cultures (at a final concentration of 100 µg/mL) and incubated for 3 hours at 37°C. The resulting formazan precipitate was dissolved in dimethylsulfoxide (Panreac) and absorbance was measured at 542 nm using a plate reader (SpectraFluor, Tecan). Data were normalized with respect to non-irradiated control values.

The evaluated conditions or groups were:
- Control cells (non-irradiated cells)
- Cells plus irradiation (at a dose of 15 or 37J/cm²)
- Non-irradiated Cells treated with H or MB
- Irradiated Cells (at a dose of 15J/cm²) treated with H or MB

### Results

The obtained results appear summarized in Figure 1 and showed that the hypericum extract by itself did not induce cell toxicity in Huh7 nor in Vero cells; neither MB (Figure 1A and 1B). The incubation of the cells with the H or with MB for 1 hour followed by light exposure, induced a slight cell toxicity (at about 10%) in Huh7 but not in Vero cells (Figure 1C and 1D). In addition, cell exposure to white light at 15 J/cm² also induced a slight cell toxicity (at about 10%) (see Figure 1E). The irradiation with 37 J/cm² induced a very high cell lethality (higher than 60% in both cell lines treated with H or MB).

### Conclusions

The hypericum extract by itself does not induce cell lethality under the evaluated conditions.

Irradiation with a dose of 15 J/cm² does not significantly affect cell viability. Irradiation with a dose of 37 J/cm² does significantly affect cell viability.

The combination of the hypericum extract with white light induces a slight cellular lethality (approximately 10%).

### Example 2. In vitro analysis of the effect of the combination of a composition comprising hypericin (hypericum extract) and light in coronavirus and herpes simplex virus type 1

### Evaluation of the photodynamic effect of the dry hypericum extract on Coronavirus and Herpes simplex type 1.

### Viruses, treatment conditions and cell infections.

- Coronavirus: HCoV-229E-GFP was obtained from HCoV-229E, RNA virus. (VR-740, American Type Culture Collection). HCoV-229E-GFP was obtained by fusing Green Fluorescence Protein (GFP) to the virus. Virus was propagated in Huh7 cells.
- Herpes simplex virus type 1: HSV-1 K26-GFP was obtained from HSV-1 K26. DNA virus. Wild type HSV-1 (F strain) (GenBank accession number for the DNA genome sequence is GU734771). HSV-1 K26-GFP was obtained by fusing GFP to the HSV-1 capsid protein VP26 recombinant virus, was propagated in Vero cells.

Both viruses incorporated the green fluorescent protein, as noted above, and, therefore, infection could be easily followed by fluorescence: infected cells showed fluorescent points in its cytoplasm in fluorescence microscopy.

The human cell lines employed were: Huh-7 hepatocarcinoma cell line (for Coronavirus) and Vero cells obtained from monkey kidney (*Cercopithecus aethiops)* (for HSV-1). Cells were cultured, as indicated before, in DMEM, supplemented with 1% (v/v) penicillin G (100 U/ml) and streptomycin (100 µg/ml) and 10% (v/v) FBS. Cells were maintained at 37°C, 5% humidity and 5% CO₂ in the incubator.

Hypericum extract (H) was as explained in example 1 and was used at two concentrations: 1 and 2 µg/ml in DMEM with 2%(v/v) FBS. Methylene blue (MB, Sigma) was also used as positive control (0.25 µg/ml in DMEM).

Virus suspensions were exposed for 30 min in solution to 100 µL of H or MB at the indicated concentrations. Then, they were exposed to white light (420-700 nm, 90 mW/cm²) (15 J/cm²), as indicated before, in the presence of H or MB and post-incubated for 30 min at room temperature. Confluence cultures performed in 96-well flasks, were infected with the treated virus suspensions at MOI of 0.5 (or also 1 were applicable for HSV-1 K26-GFP) for 1 hour. Infection was performed in DMEM without FBS. After infection, cells were washed three times with PBS and post-incubated for 24 hours or 48 hours before evaluating the infection ability directly by fluorescence and contrast phase microscopy and by flow cytometry. In the latter case, 24 hours after infection, cells were trypsinized and evaluated by flow cytometry. For that, cultures were dissociated by 1 minute incubation with 0.05% (v/v) trypsin/0.1% (v/v) EDTA (Invitrogen) at room temperature, washed and fixed in 4% (v/v) paraformaldehyde for 15 minutes. Subsequently, cells were rinsed and resuspended in PBS. Analysis was performed using a FACSalibour Flow Cytometer (BD Biosciences).

The evaluated conditions or groups were:
- Non-infected cells.
- Infected cells with non-treated and non-irradiated virus.
- Virus-infected cells wherein the virus was exposed to H or MB, without irradiation.
- Virus-infected cells wherein the virus was irradiated (15 J/cm²).
- Virus-infected cells wherein the virus was treated with H or MB plus irradiation (15 J/cm²).

### Microscopic observation

Microscopic observations were performed using a fluorescence microscope (BX-61, Olympus) with the blue filter sets (450-490 nm, exciting filter BP 490). Images were obtained with an Olympus CCD DP70 digital camera. Fluorescence intensity analysis (GFP fluorescence) was performed using imaged version 1.52a.

### Results of infection by Coronavirus

Figure 2 shows the results obtained in this example for HCoV-229E-GFP. Images shown in Figure 2 illustrate that hypericum extract by itself exhibited a low viricidal ability on Coronavirus: 20% lower ability of infection if the virus was exposed to H at 2 µg/mL (but not H at 1 µg/mL) prior to Huh7 cell infection (see Figure 2A). 15 J/cm² of white light exposure prior to cell infection did not inhibit cell infection. Virus exposed to a combination of H at 1 µg/mL and 15 J/cm² of white light showed a reduction in viral infectivity (10% of reduction approximately). In addition, surprisingly, the combination of H at 2 µg/mL and light (white light at 15 J/cm²) induced about 90% of cell infection inhibition (Infection 70% lower than irradiated virus and 90% lower than non-irradiated virus).

The obtained results observed in Figure 2 were confirmed by flow cytometry (Figure 3). Huh7 cells infected by coronavirus HCoV-229E-GFP at the MOI previously mentioned of 0.5, incubated with MB (0,25 µg/ml) or H (2 µg/ml) and not irradiated did not show any differences with respect to cells infected with untreated virus, indicating that, under these treatment conditions, H and MB did not affect the ability of virus infection. However, when viruses were subjected to H or MB followed by white light (420-700 nm, 90 mW/cm²) exposure a great decrease in the cellular infection was detected by flow cytometry, supporting the observations performed in the fluorescence microscope.

### Results of infection by Herpes simplex virus type 1

Figure 4 shows the results obtained in this example for HSV-1 K26-GFP. Figure 4 shows no substantial differences on the viricidal effects of H in the different conditions tested, under infection conditions of MOI 0.5 (MOI of 1 was also tested with the same results).

Vero cells showed a high HSV-1 K26-GFP infection. There was not a viricidal effect of H by itself at the tested concentrations. The combination of H plus white light (420-700 nm, 90 mW/cm²) induced a low viricidal effect on HSV-1 K26. On the contrary MB induced a high inhibition of infection by itself and particularly in combination with white light.

### Conclusions

Irradiation affects the infective capacity of the HCoV-229E-GFP but not of HSV-1 K26-GFP.

H does not have a viricidal effect against HSV-1 K26-GFP at either of the two concentrations used.

The H extract by itself has a slight viricidal effect on the HCoV-229E-GFP but not on HSV-1 K26-GFP as noted above.

The combination of H and white light showed a synergistic effect with a surprisingly increased inhibition of the viral infection in HCoV-229E-GFP at 2 µg/mL. This synergistic effect was not seen in HSV-1 K26-GFP.

The results of examples 1 and 2 included above have been obtained Coronavirus HCoV-229E, a recognized model for the analysis of Coronavirus, and therefore, a person skilled in the art would recognize that the results obtained for said Coronavirus are easily extrapolated to other Coronavirus due to their structural and genetic similarities. Hence, the results obtained for Coronavirus HCoV-229E are directly applicable to Coronavirus SARS-CoV-2.

## Claims

1. Composition comprising hypericin for use in the prevention and/or treatment of a coronavirus disease wherein the composition is used in combination with light, wherein the dose of light is between 10 and 36 J/cm², and wherein the concentration of hypericin is between 0.003 µg/mL and 0.02 µg/mL.

2. Composition for use accordance with claim 1, **characterized in that** the composition comprising hypericin is applied on the skin of a subject and after its application is exposed to light.

3. Composition for use in accordance with any one of claims 1 or 2, **characterized in that** the light comprises visible light.

4. Composition for use in accordance with claim 3, **characterized in that** the visible light is selected from blue light (420-495 nm), amber light (550-580 nm), white light (420-700 nm).

5. Composition for use in accordance with any one of claims 1 to 4, **characterized in that** the coronavirus disease is a disease caused by Coronavirus HCoV-229E or Coronavirus SARS-CoV-2.

6. Composition for use in accordance with any one of claims 1 to 5, **characterized in that** the coronavirus disease is COVID-19.

7. Composition for use in accordance with any one of claims 1 to 6, **characterized in that** the hypericin is provided in the composition in the form of an hypericum extract which comprises hypericin.

8. Composition for use in accordance with claim 7, **characterized in that** the concentration of hypericum extract is 2 µg/mL.

9. Use of a composition comprising hypericin for the inactivation of coronavirus from an inert surface, or for the prevention of viral contamination of an inert surface, wherein the composition is applied on said inert surface and after its application is exposed to light,
wherein the dose of light is between 10 and 36 J/cm² , and
wherein the concentration of hypericin is between 0.003 µg/mL and 0.02 µg/mL.

10. Method for the inactivation of coronavirus from an inert surface, or for the prevention of viral contamination of an inert surface by coronavirus comprising:
a) application of a composition comprising hypericin to the inert surface; and
b) exposing said inert surface with the composition to light
wherein the dose of light is between 10 and 36 J/cm², and
wherein the concentration of hypericin is between 0.003 µg/mL and 0.02 µg/mL.

11. Use of a composition comprising hypericin for the inactivation of coronavirus from a liquid, or for the prevention of viral contamination of a liquid by coronavirus, wherein the composition is applied on said liquid and after its application is exposed to light and wherein the use is ex vivo or in vitro
wherein the dose of light is between 10 and 36 J/cm², and
wherein the concentration of hypericin is between 0.003 µg/mL and 0.02 µg/mL.

12. Use in accordance with claim 11, **characterized in that** the liquid is blood or plasma.

13. Method for the inactivation of coronavirus from a liquid, or for the prevention of viral contamination of a liquid by coronavirus comprising:
a) application of a composition comprising hypericin to the liquid; and
b) exposing said liquid with the composition to light,
wherein the method is carried out ex vivo or in vitro
wherein the dose of light is between 10 and 36 J/cm² , and
wherein the concentration of hypericin is between 0.003 µg/mL and 0.02 µg/mL.

## Patentansprüche

1. Zusammensetzung, die Hypericin umfasst, zur Verwendung bei der Vorbeugung und/oder Behandlung einer Coronavirus-Krankheit, wobei die Zusammensetzung in Kombination mit Licht verwendet wird,
wobei die Lichtdosis zwischen 10 und 36 J/cm² liegt und
wobei die Konzentration von Hypericin zwischen 0,003 µg/mL und 0,02 µg/mL liegt.

2. Zusammensetzung zur Verwendung nach Anspruch 1, **dadurch gekennzeichnet, dass** die Zusammensetzung, die Hypericin umfasst, auf die Haut eines Individuums aufgetragen wird und nach dem Auftragen derselben Licht ausgesetzt wird.

3. Zusammensetzung zur Verwendung nach einem der Ansprüche 1 oder 2, **dadurch gekennzeichnet, dass** das Licht sichtbares Licht umfasst.

4. Zusammensetzung zur Verwendung nach Anspruch 3, **dadurch gekennzeichnet, dass** das sichtbare Licht aus blauem Licht (420-495 nm), bernsteinfarbenem Licht (550-580 nm) und weißem Licht (420-700 nm) ausgewählt wird.

5. Zusammensetzung zur Verwendung nach einem der Ansprüche 1 bis 4, **dadurch gekennzeichnet, dass** die Coronavirus-Krankheit eine Krankheit ist, die durch Coronavirus HCoV-229E oder Coronavirus SARS-CoV-2 verursacht wird.

6. Zusammensetzung zur Verwendung nach einem der Ansprüche 1 bis 5, **dadurch gekennzeichnet, dass** die Coronavirus-Krankheit COVID-19 ist.

7. Zusammensetzung zur Verwendung nach einem der Ansprüche 1 bis 6, **dadurch gekennzeichnet, dass** das Hypericin in der Zusammensetzung in Form eines Hypericumextrakts bereitgestellt wird, der Hypericin umfasst.

8. Zusammensetzung zur Verwendung nach Anspruch 7, **dadurch gekennzeichnet, dass** die Konzentration des Hypericumextrakts 2 µg/mL beträgt.

9. Verwendung einer Zusammensetzung, die Hypericin umfasst, zur Inaktivierung von Coronavirus von einer inerten Oberfläche oder zur Vorbeugung von viraler Verunreinigung einer inerten Oberfläche, wobei die Zusammensetzung auf die inerte Oberfläche aufgetragen wird und nach dem Auftragen derselben Licht ausgesetzt wird,
wobei die Lichtdosis zwischen 10 und 36 J/cm² liegt und
wobei die Konzentration von Hypericin zwischen 0,003 µg/mL und 0,02 µg/mL liegt.

10. Verfahren zur Inaktivierung von Coronavirus von einer inerten Oberfläche oder zur Vorbeugung von viraler Verunreinigung einer inerten Oberfläche durch Coronavirus, umfassend:
a) Auftragen einer Zusammensetzung, die Hypericin umfasst, auf die inerte Oberfläche; und
b) Aussetzen der inerten Oberfläche mit der Zusammensetzung gegenüber Licht,
wobei die Lichtdosis zwischen 10 und 36 J/cm² liegt und
wobei die Konzentration von Hypericin zwischen 0,003 µg/mL und 0,02 µg/mL liegt.

11. Verwendung einer Zusammensetzung, die Hypericin umfasst, zur Inaktivierung von Coronavirus aus einer Flüssigkeit oder zur Vorbeugung von viraler Verunreinigung einer Flüssigkeit durch Coronavirus,
wobei die Zusammensetzung auf die Flüssigkeit aufgetragen und nach dem Auftragen derselben Licht ausgesetzt wird und wobei die Verwendung *ex vivo* oder *in vitro* erfolgt,
wobei die Lichtdosis zwischen 10 und 36 J/cm² liegt und
wobei die Konzentration von Hypericin zwischen 0,003 µg/mL und 0,02 µg/mL liegt.

12. Verwendung nach Anspruch 11, **dadurch gekennzeichnet, dass** die Flüssigkeit Blut oder Plasma ist.

13. Verfahren zur Inaktivierung von Coronavirus aus einer Flüssigkeit oder zur Vorbeugung von viraler Verunreinigung einer Flüssigkeit durch Coronavirus, umfassend:
a) Auftragen einer Zusammensetzung, die Hypericin umfasst, auf die Flüssigkeit; und
b) Aussetzen der Flüssigkeit mit der Zusammensetzung gegenüber Licht,
wobei das Verfahren *ex vivo* oder *in vitro* erfolgt,
wobei die Lichtdosis zwischen 10 und 36 J/cm² liegt und
wobei die Konzentration von Hypericin zwischen 0,003 µg/mL und 0,02 µg/mL liegt.

## Revendications

1. Composition comprenant de l'hypéricine pour la prévention et/ou le traitement d'une maladie à coronavirus, la composition étant utilisée en combinaison avec de la lumière,
dans lequel la dose de lumière est comprise entre 10 et 36 J/cm², et
dans lequel la concentration d'hypéricine est comprise entre 0,003 µg/mL et 0,02 µg/mL.

2. Composition à utiliser selon la revendication 1, **caractérisée en ce que** la composition comprenant de l'hypéricine est appliquée sur la peau d'un sujet et qu'après son application, elle est exposée à la lumière.

3. Composition pour utilisation selon l'une des revendications 1 ou 2, **caractérisée en ce que** la lumière comprend la lumière visible.

4. Composition à utiliser conformément à la revendication 3, **caractérisée en ce que** la lumière visible est choisie parmi la lumière bleue (420-495 nm), la lumière ambre (550-580 nm), la lumière blanche (420-700 nm).

5. Composition pour utilisation selon l'une des revendications 1 à 4, **caractérisée en ce que** la maladie à coronavirus est une maladie causée par le coronavirus HCoV-229E ou le coronavirus SARS-CoV-2.

6. Composition utilisable selon l'une des revendications 1 à 5, **caractérisée en ce que** le coronavirus est le COVID-19.

7. Composition pour utilisation selon l'une quelconque des revendications 1 à 6, **caractérisée en ce que** l'hypéricine est apportée dans la composition sous la forme d'un extrait d'hypericum qui comprend de l'hypéricine.

8. Composition pour utilisation selon la revendication 7, **caractérisée en ce que** la concentration de l'extrait d'hypericum est de 2 µg/mL.

9. Utilisation d'une composition comprenant de l'hypéricine pour inactiver le coronavirus d'une surface inerte ou pour prévenir la contamination virale d'une surface inerte, dans laquelle la composition est appliquée sur ladite surface inerte et, après son application, est exposée à la lumière,
dans lequel la dose de lumière est comprise entre 10 et 36 J/cm², et
dans lequel la concentration d'hypéricine est comprise entre 0,003 µg/mL et 0,02 µg/mL.

10. Procédé d'inactivation du coronavirus d'une surface inerte ou de prévention de la contamination virale d'une surface inerte par le coronavirus comprenant :
a) l'application d'une composition comprenant de l'hypericine sur la surface inerte ; et
b) l'exposition à la lumière de ladite surface inerte contenant la composition
dans lequel la dose de lumière est comprise entre 10 et 36 J/cm², et
dans lequel la concentration d'hypéricine est comprise entre 0,003 µg/mL et 0,02 µg/mL.

11. Utilisation d'une composition comprenant de l'hypéricine pour inactiver le coronavirus d'un liquide ou pour prévenir la contamination virale d'un liquide par le coronavirus,
dans lequel la composition est appliquée sur ledit liquide et, après son application, est exposée à la lumière et dans lequel l'utilisation est ex vivo ou in vitro
dans lequel la dose de lumière est comprise entre 10 et 36 J/cm², et
dans lequel la concentration d'hypéricine est comprise entre 0,003 µg/mL et 0,02 µg/mL.

12. Utilisation selon la revendication 11, **caractérisée en ce que** le liquide est du sang ou du plasma.

13. Procédé d'inactivation du coronavirus dans un liquide ou de prévention de la contamination virale d'un liquide par le coronavirus comprenant :
a) l'application d'une composition comprenant de l'hypericine sur le liquide ; et
b) l'exposition dudit liquide contenant la composition à la lumière,
dans lequel la méthode est appliquée ex vivo ou in vitro
dans lequel la dose de lumière est comprise entre 10 et 36 J/cm², et
dans lequel la concentration d'hypéricine est comprise entre 0,003 µg/mL et 0,02 µg/mL.
